Europäisches Patentamt

European Patent Office

Office européen des brevets

(19)

(11) Publication number: **0 026 244**
**B1**

(12) **EUROPEAN PATENT SPECIFICATION**

(45) Date of publication of patent specification: **19.10.83**

(51) Int. Cl.³: **C 07 C 51/27, C 07 C 63/00**

(21) Application number: **79302003.3**

(22) Date of filing: **26.09.79**

(54) **Preparation of a mixture of polycylic aromatic polycarboxylic acids soluble in acetone, but insoluble in water.**

(43) Date of publication of application:
**08.04.81 Bulletin 81/14**

(45) Publication of the grant of the patent:
**19.10.83 Bulletin 83/42**

(84) Designated Contracting States:
**AT BE CH DE FR GB IT LU NL SE**

(56) References cited:
**DE - B - 1 133 714**
**DE - C - 743 225**
**GB - A - 797 726**
**US - A - 2 555 410**
**US - 3 209 024**
**US - A - 4 137 418**

(73) Proprietor: **GULF RESEARCH & DEVELOPMENT COMPANY**
**P.O. Box 2038**
**Pittsburgh Pennsylvania 15230 (US)**

(72) Inventor: **Schulz, Johann Gustav D**
**201 Conover Road**
**Pittsburgh, Pennsylvania 15208 (US)**
Inventor: **Sabourin, Edward T.**
**4324 Winchester Drive**
**Allison Park, Pennsylvania 15101 (US)**

(74) Representative: **Huskisson, Frank Mackie**
**48 St. Vincent Street**
**Glasgow, G2 5TT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

**0 026 244**

Preparation of a mixture of polycyclic aromatic polycarboxylic
acids soluble in acetone, but insoluble in water

Background of the Invention
*1. Field of the Invention*
This invention relates to a process for preparing a mixture of polycyclic aromatic polycarboxylic acids that is substantially soluble in acetone but substantially insoluble in water which comprises subjecting a slurry containing coal to reaction with aqueous nitric acid in an atmosphere containing molecular oxygen, mechanically separating the solids in the resulting slurry, extracting the resulting solids with a polar solvent to separate therefrom a mixture of polycyclic aromatic polycarboxylic acids substantially soluble in acetone but substantially insoluble in water and a mixture of polycyclic aromatic polycarboxylic acids substantially insoluble in acetone and substantially insoluble in water.

*2. Description of the Prior Art*
In our U.S. Patent No. 4,052,448 we disclosed a process for preparing a mixture of polycyclic aromatic polycarboxylic acids that is substantially soluble in acetone but substantially insoluble in water which involved subjecting a slurry containing coal to reaction with aqueous nitric acid, mechanically separating the solids in the resulting slurry, extracting the resulting solids with a polar solvent and then separating the polar solvent from the extract to recover the mixture of polycyclic aromatic polycarboxylic acids substantially soluble in acetone but substantially insoluble in water.

U.S.—A—2,555,410 describes the nitric acid oxidation of coal tar to produce water soluble acids. By carrying out the oxidation in the presence of oxygen at a partial pressure of from 100 to 1000 pounds per square inch (6.895 × 10⁵ to 6.894 to 10⁶ Pa) the amount of acid required and the degree of oxidation to carbon dioxide are said to be reduced compared with conventional processes of this type.

GB—A—797,725 also describes the oxidation of carbonaceous material with nitric acid to produce products which are wholly soluble in water. The water-soluble products are further processed by heating at above 100°C to render higher molecular weight materials insoluble, the lower molecular weight products being soluble and extracted with water or polar solvent.

Summary of the Invention
We have now found, unexpectedly, that we can greatly increase the amount of acetone-soluble, water-insoluble polycyclic aromatic polycarboxylic acids obtained in the process disclosed and claimed in said U.S. Patent No. 4,052,448 if we carry out the nitric acid reaction therein in an atmosphere containing molecular oxygen.

The individual components of the acetone-soluble, water-insoluble polycyclic aromatic polycarboxylic acids obtained herein are believed to be composed of condensed and/or non-condensed benzene rings, with an average number of benzene rings in the individual molecules ranging from two to 10, but generally from three to eight. On the average, the number of carboxyl groups carried by the individual molecules will range from four to 10, generally from six to eight and the average number of nitro groups from one to four, generally from two to three. The average molecular weight of the mixture will range from 600 to 1500, generally from 700 to 1000, and the average neutral equivalent will range from 80 to 200, generally from 100 to 150. A typical analysis of the desired mixture is defined below in Table I in approximate amounts.

TABLE I

| | Weight Per Cent | |
|---|---|---|
| | Broad Range | Preferred Range |
| Carbon | 50 to 60 | 52 to 56 |
| Hydrogen | 3 to 5 | 3.7 to 4.4 |
| Nitrogen | 3 to 6 | 4 to 5 |
| Oxygen | 25 to 45 | 30 to 40 |
| Sulfur | 0.2 to 0.5 | 0.3 to 0.5 |
| Ash | 0.1 to 5 | 0.3 to 3 |

In the preferred procedure herein there is introduced into a reactor an aqueous solution of nitric acid and a carbonaceous material. The nitric acid can have a concentration of 5 to 90 per cent, but

2

preferably will be in the range of 10 to 70 per cent. The carbonaceous material is preferably a solid in the form of a slurry, for example, an aqueous slurring containing the carbonaceous material in particulate form and from 50 to 90 weight per cent of water.

The solid carbonaceous material that can be used herein can have the following composition on a moisture-free basis:

TABLE II

| | Weight Per Cent | |
| --- | --- | --- |
| | Broad Range | Preferred Range |
| Carbon | 45 to 95 | 60 to 92 |
| Hydrogen | 2.5 to 7 | 4 to 6 |
| Oxygen | 2.0 to 45 | 3 to 25 |
| Nitrogen | 0.75 to 2.5 | 0.75 to 2.5 |
| Sulfur | 0.3 to 10 | 0.5 to 6 |

The carbon and hydrogen content of the carbonaceous material will reside primarily in multi-ring aromatic compounds (condensed and/or uncondensed), heterocyclic compounds, etc. Oxygen and nitrogen are believed to be present primarily in chemical combination. Some of the sulfur is believed to be present in chemical combination with the aromatic compounds and some in chemical combination with inorganic elements associated therewith, for example, iron and calcium.

In addition to the above the solid carbonaceous material being treated herein will also contain solid, primarily inorganic, compounds which will not be converted to the desired organic mixture claimed herein, which are termed ash and are composed chiefly of compounds of silicon, aluminum, iron and calcium, with smaller amounts of compounds of magnesium, titanium, sodium and potassium. The ash content of the carbonaceous material treated herein will amount to less than 50 weight per cent, based on the moisture-free carbonaceous material, but, in general, will amount to 0.1 to 30 weight per cent, usually 0.5 to 20 weight per cent.

Anthracitic, bituminous and subbituminous coal, lignitic materials, and other types of coal products referred to in ASTM D-388 are exemplary of the solid carbonaceous materials which can be treated in accordance with the process defined herein to produce the claimed organic mixture. Some of these carbonaceous materials in their raw state will contain relatively large amounts of water. These can be dried prior to use herein. The carbonaceous material, prior to use, is preferably ground in a suitable attrition machine, such as a hammermill, to a size such that at least 50 per cent of the carbonaceous material will pass through a 40-mesh (U.S. Series) sieve. As noted, the carbonaceous material is slurried in a suitable carrier, preferably water, prior to reaction with nitric acid. If desired, the carbonaceous material can be treated, prior to reaction herein, using any conventional means, to remove therefrom any materials forming a part thereof that will not be converted in reaction with nitric acid herein.

The reactant mixture in the reactor is stirred while being maintained at a temperature of 15° to 200°C, preferably 50° to 100°C, and a molecular oxygen pressure of 14.7 to 1470 pounds per square inch gauge ($9.8 \times 104$ to $9.8 \times 10^6$ Pa), preferably 73.5 to 735 pounds per square inch gauge ($4.9 \times 10^5$ to $4.9 \times 10^6$ Pa) for 0.5 to 15 hours, preferably two to six hours. In order to obtain the desired mixture herein without losing appreciable amounts of carboxyl groups on the acids that are formed during the oxidation, and to obtain the desired acids in high yields in the reactor, it is absolutely critical that the reaction conditions therein, namely nitric acid concentration, temperature, pressure of molecular oxygen and reaction time, be so correlated to minimize and, preferably, to avoid decarboxylation. Gaseous products, such as nitrogen oxides, if any, can be removed from the reaction zone.

The reaction product is removed from the reactor in any convenient manner. We have found that the reaction product is soluble in, or reactable with, sodium hydroxide. At this point it is necessary to separate the oxidized product from the water and nitric acid associated therewith. This separation must be accomplished in a manner so that the carboxyl groups are not removed from the acid product. Distillation for the removal of water will not suffice, because under the conditions required for such separation, a significant loss of carboxyl groups would occur. Accordingly, we have found that a mechanical separation will suffice. The reaction product is therefore led to a first separator which can be, for example, a filter or a centrifuge.

The solids that are recovered in the first separator, also soluble in sodium hydroxide, are led to a second separator wherein they are subjected to extraction with acetone that is introduced therein. Such separation can be carried out at a temperature of 20° to 60°C, preferably 25° to 50°C, and a pressure of atmospheric to 500 pounds per square inch gauge (atmospheric to $3.43 \times 10^6$ Pa), preferably atmospheric to 100 pounds per square inch gauge (atmospheric to $6.86 \times 10^5$ Pa). The solid material, insoluble in acetone, is removed from the second separator by one line and the acetone solution of the desired acid mixture by another line. The acetone solution is then led to a drier or evaporator wherein acetone is separated therefrom by one line and the desired acetone-soluble, water-insoluble polyaromatic, polycarboxylic acid mixture prepared herein is recovered by another line. As before, the acid mixture in the drier must be treated by so correlating the conditions therein to remove acetone therefrom in such manner so as to minimize and, preferably, avoid, decarboxylation. The temperature can be in the range of 10° to 60°C, preferably 20° to 50°C, the pressure $1.31 \times 10^3$ Pa to atmospheric, preferably $3.95 \times 10^3$ Pa to atmospheric, for 0.5 to 24 hours, preferably about one to about five hours.

The filtrate obtained in the first separator is removed therefrom and in all cases will contain water, nitric acid and most of the inorganic material (ash) that was present in the carbonaceous charge. In addition there can also be present other oxidized material, which are primarily acetone-soluble, water-soluble organic acids.

Separation of the filtrate into its component parts can be effected as follows. It can be passed to a distillation tower maintained at a temperature of 50° to 100°C, preferably 70° to 90°C, and a pressure of $1.31 \times 10^3$ Pa to atmospheric, preferably $3.95 \times 10^3$ Pa to atmospheric. Under these conditions nitric acid and water are removed from the distillation tower by one line and solids by another line. The solids are led to a third separator where they are subjected to extraction with acetone introduced therein. The conditions in the third separator are similar to those used in the second separator. A mixture of acetone-soluble, water-soluble organic acids is removed from the third separator by one line and substantially all of the inorganic material that was present in the carbonaceous charge by another line.

Several runs were carried out in which a North Dakota Lignite analyzing as follows, on a substantially moisture-free basis, was subjected to oxidation: 65.03 weight per cent carbon, 4.0 weight per cent hydrogen, 27.0 weight per cent oxygen, 0.92 weight per cent sulfur, 0.42 weight per cent nitrogen and 0.04 weight per cent moisture. The ash was further analyzed and found to contain 43 weight per cent oxygen, 7.8 weight per cent sulfur and the remainder metals. In each of Runs Nos. 1, 2 and 3, 70 per cent aqueous nitric acid was gradually added over a period of two hours to a stirred slurry maintained at 70°C containing 800 grams of powdered lignite defined above (corresponding to 540 grams of moisture-free feed) and 600 grams of water. In each of these runs the pressure in the closed reactor at the beginning was atmospheric and was permitted to rise during the course of the reaction to its autogeneous pressure. In Run No. 4, otherwise similar to Runs Nos. 1, 2 and 3, molecular oxygen was also continuously introduced into the reactor to maintain therein a pressure of 500 pounds per square inch gauge ($3.33 \times 10^6$ Pa) over the course of the reaction.

At the end of the reaction period the product slurry was withdrawn from the reaction zone and filtered to obtain a solids fraction and a filtrate. The solids were extracted with acetone at atmospheric temperature and pressure. The acetone solution was then subjected to evaporation at atmospheric temperature and pressure to obtain the desired mixture herein. The acetone insoluble portion was found to be soluble in sodium hydroxide and to comprise organic acids of a relatively higher molecular weight than the acetone-soluble portion.

The work-up of the filtrate was carried out as follows. Initially the filtrate was subjected to distillation to separate unreacted nitric acid and water therefrom. The remaining solids were subjected to extraction with acetone at atmospheric temperature and atmospheric pressure. The acetone solution was dried to remove acetone therefrom, resulting in the recovery of small amounts of the acetone-soluble, water-soluble organic acids substantially completely soluble in sodium hydroxide. The residue was mainly ash. The data obtained are summarized below in Table III.

TABLE III
Weight Distribution of Polycyclic Aromatic Polycarboxylic Acids

| Run No. | Total Nitric Acid Added Grams* | Acetone-Insoluble Water-Insoluble Acids Grams | Acetone-Soluble Water-Insoluble Acids Grams | Acetone-Soluble Water-Soluble Acids Grams | Total Aromatic Acids Produced Grams | Total Nitric Acid Consumed Grams |
|---|---|---|---|---|---|---|
| 1 | — | 302.9 | 142.5 | 104.0 | 549.4 | 566.9 |
| 2 | — | 107.0 | 156.0 | 174.0 | 437.0 | 872.0 |
| 3 | — | 86.0 | 109.0 | 200.0 | 395.0 | 1050.0 |
| 4 | — | 154.7 | 224.7 | 192.0 | 571.4 | 453.0 |

* On a 100 per cent basis.

The results obtained above are most unusual. Doubling the amount of nitric acid in Run No. 2 over that used in Run No. 1 might have led one to expect an increase in the amount of acetone-soluble, water-insoluble acids obtained, since the latter are believed to be a higher oxidation species of the acetone-insoluble, water-insoluble species, but the amount of increase was slight and at the expense of an increased nitric acid consumption. Further increase in the amount of nitric acid introduced into the reaction zone in Run No. 3, unfortunately, greatly reduced the amount of acetone-soluble, water-insoluble acids produced, with a still greater consumption of nitric acid. However, when Run No. 1 was repeated, except that the process was conducted in an atmosphere containing molecular oxygen, the amount of acetone-soluble, water-insoluble acids produced was greatly increased with a greatly decreased nitric acid consumption.

Although we have stated above that the desired composition is acetone-soluble and we have shown the use of acetone as suitable in the process defined herein, this has been done merely as a characterization of the composition and to exemplify one embodiment of our process. Many polar solvents alone, or polar solvents in combination with other solvents, can be used in place of acetone herein. Among the polar solvents, or combination of solvents, that can be used are methanol, ethanol, isopropanol, methyl ethyl ketone, tetrahydrofuran, dioxane, cyclohexanone, tetrahydrofurfuryl alcohol, acetone in combination with methanol, methyl ethyl ketone in combination with methanol, isopropanol or water, tetrahydrofuran in combination with methanol or water, dioxane in combination with methanol or water.

**Claims**

1. A process for preparing a mixture of polycyclic aromatic polycarboxylic acids that is substantially soluble in acetone but substantially insoluble in water which comprises subjecting a slurry containing coal to reaction with aqueous nitric acid in an atmosphere containing molecular oxygen, mechanically separating the solids in the resulting slurry, extracting the resulting solids with a polar solvent to separate therefrom a mixture of polycyclic aromatic polycarboxylic acids substantially soluble in acetone but substantially insoluble in water and a mixture of polycyclic aromatic polycarboxylic acids substantially insoluble in acetone and substantially insoluble in water, wherein the aqueous nitric acid has a concentration of from 5 to 90 per cent and the reaction is carried out at a temperature of from 15° to 200°C and a pressure of molecular oxygen of from 14.7 to 1470 pounds per square inch gauge ($9.8 \times 10^4$ to $9.8 \times 10^6$ Pa) for a period of from 0.5 to 14 hours.

2. A process as claimed in claim 1 wherein the aqueous nitric acid has a concentration of from 10 to 70 per cent and the reaction is carried out at a temperature of from 50° to 100°C and a pressure of molecular oxygen of from 73.5 to 735 pounds per square inch gauge ($4.9 \times 10^5$ to $4.9 \times 10^6$ Pa) for from two to six hours.

3. A process as claimed in claim 1 wherein said polar solvent is acetone.

4. A process as claimed in claim 1 wherein the mechanical separation is effected by filtration.

5. A process as claimed in claim 1 wherein the acetone is separated from the extract by drying.

6. A process as claimed in claim 1 wherein the coal is lignite.

**Patentansprüche**

1. Ein Verfahren zur Herstellung eines Gemisches von polycyclischen aromatischen Polycarbonsäuren, das im wesentlichen in Aceton löslich, aber im wesentlichen in Wasser unlöslich ist, gekennzeichnet durch die Reaktion einer kohlehaltigen Aufschlämmung mit wässeriger Salpetersäure in einer Atmosphäre, die molekularen Sauerstoff enthält, mechanische Abtrennung der Feststoffe aus der hieraus resultierenden Aufschlämmung, Extraktion der so erhaltenen Feststoffe mit Hilfe eines polaren Lösungsmittels, um daraus ein Gemisch von polycyclischen aromatischen Polycarbonsäuren, das im wesentlichen in Aceton löslich, aber im wesentlichen in Wasser unlöslich ist, und ein Gemisch von polycyclischen aromatischen Polycarbonsäuren, das im wesentlichen in Aceton unlöslich und in Wasser unlöslich ist, abzutrennen, wobei die wässerige Salpetersäure eine Konzentration von 5 bis 90 % aufweist und die Reaktion bei einer Temperatur von 15 bis 200° C und bei einem Druck des molekularen Sauerstoffes von 14,7 bis 1470 pounds per square inch gauge ($9,8 \times 10^4$ bis $9,8 \times 10^6$ Pa) während eines Zeitraumes von 0,5 bis 15 h ausgeführt wird.

2. Verfahren nach Anspruch 1, wobei die wässerige Salpetersäure eine Konzentration von 10 bis 70 % aufweist und die Reaktion bei einer Temperatur von 50 bis 100° C und einem Druck des molekularen Sauerstoffes von 73,5 bis 735 pounds per square inch gauge ($4,9 \times 10^5$ bis $4,9 \times 10^6$ Pa) während 2 bis 6 h ausgeführt wird.

3. Verfahren nach Anspruch 1, wobei das genannte polare Lösungsmittel Aceton ist.

4. Verfahren nach Anspruch 1, wobei die mechanische Abtrennung durch Filtrieren erfolgt.

5. Verfahren nach Anspruch 1, wobei das Aceton durch Trocknen von dem Extrakt getrennt wird.

6. Verfahren nach Anspruch 1, wobei die Kohle Lignit ist.

**Revendications**

1. Procédé pour préparer un mélange d'acides polycarboxyliques aromatiques polycycliques, qui est essentiellement soluble dans l'acétone mais essentiellement insoluble dans l'eau, procédé qui consiste à soumettre une suspension, contenant du charbon, à une réaction avec de l'acide nitrique aqueux dans une atmosphère contenant de l'oxygène moléculaire, à séparer mécaniquement les solides présents dans la suspension résultante, à extraire à l'aide d'un solvant polaire les solides résultants pour en séparer un mélange d'acides polycarboxyliques aromatiques polycycliques essentiellement soluble dans l'acétone mais essentiellement insoluble dans l'eau et un mélange d'acides polycarboxyliques aromatiques polycycliques essentiellement insoluble dans l'acétone et essentiellement insoluble dans l'eau, l'acide nitrique aqueux ayant une concentration de 5 à 90 % et la réaction étant effectuée à une température comprise entre 15° et 200° C et à une presssion manométrique d'oxygène moléculaire comprise entre 14,7 et 1470 livres par pouce carré ($9,8 \times 10^4$ à $9,8 \times 10^6$ Pa) pendant une période d'une demi-heure à 15 heures.

2. Procédé selon la revendication 1, dans lequel l'acide nitrique aqueux a une concentration de 10 à 70 %, et la réaction est effectuée à une température de 50° à 100° C et à une pression manométrique d'oxygène moléculaire de 73,5 à 735 livres par pouce carré ($4,9 \times 10^5$ à $4,9 \times 10^6$ Pa) durant deux à six heures.

3. Procédé selon la revendication 1, dans lequel ledit solvant polaire est l'acétone.

4. Procédé selon la revendication 1, dans lequel la séparation mécanique est effectuée par filtration.

5. Procédé selon la revendication 1, dans lequel l'acétone est séparée de l'extrait par séchage.

6. Procédé selon la revendication 1, dans lequel le charbon est du lignite.